# EUROPEAN PATENT SPECIFICATION

(11) **EP 1 337 199 B1**
(45) Date of publication and mention of the grant of the patent: **07.02.2007**
(21) Application number: 01977377.9
(22) Date of filing: 02.10.2001
(51) Int. Cl.: A61F 2/84

(54) **BODY SOCK FOR A STENT DELIVERY CATHETER**
STRUMPFMANSCHETTE FÜR STENTEINFÜHRUNGSKATHETER
MANCHON POUR CORPS D'EXTENSEUR

(30) Priority: 05.10.2000 US 238795 P
(43) Date of publication of application: 27.08.2003
(73) Proprietor: Boston Scientific Limited, St. Michael, Barbados, West Indies (BB)
(72) Inventor: SEPPALA, Jan, Maple Grove, MN 55369 (US); DICAPRIO, Fernando, St. Paul, MN 55105 (US); RIZQ, Raed, Fridley, MN 55432 (US)
(74) Representative: Graalfs, Edo
(86) International application number: PCT/US2001/030853
(87) International publication number: WO 2002/028317

(56) References cited:
- WO-A-01/22903
- WO-A-01/80780
- WO-A1-00/76425
- US-A- 5 108 416
- US-A- 5 158 548
- US-A- 5 944 726

## Description

It is well understood that stents which are not properly secured or retained to the catheter may slip and either be lost or be deployed in the wrong location or partially deployed. Traditionally, in order to provide proper securement of the stent on the catheter; the stent is crimped to a predetermined area of the catheter.

In the past, crimping has been done by hand or by a crimping apparatus, often resulting in the application of undesired uneven forces to the stent. Such a stent must either be discarded or re-crimped. Stents which have been crimped multiple times can suffer from fatigue and may be scored or otherwise marked which can cause thrombosis. A poorly crimped stent can also damage the underlying balloon.

Stents and stent delivery assemblies are utilized in a number of medical procedures and situations, and as such their structure and function are well known. A stent is a generally cylindrical prosthesis introduced via a catheter into a lumen of a body vessel in a configuration having a generally reduced diameter and then expanded to the diameter of the vessel. In its expanded configuration, the stent supports and reinforces the vessel walls while maintaining the vessel in an open, unobstructed condition.

The present invention avoids these problems by providing stent retaining sock(s) or sleeves which are capable of securing a stent to the catheter without the need to crimp the stent into place. The sock(s) may be utilized with nearly any type of stent. Both self-expanding and inflation expandable stents are well known and widely available in a variety of designs and configurations. Self-expanding stents must be maintained under a contained sheath or sock(s) in order to maintain their reduced diameter configuration during delivery of the stent to its deployment site. Inflation expandable stents are crimped to their reduced diameter about the delivery catheter, then maneuvered to the deployment site and expanded to the vessel diameter by fluid inflation of a balloon positioned between the stent and the delivery catheter. The present invention is particularly concerned with delivery and deployment of inflation expandable stents, although it is generally applicable to self-expanding stents when used with balloon catheters.

In advancing an inflation expandable stent through a body vessel to the deployment site, there are a number of important considerations. The stent must be able to securely maintain its axial position on the delivery catheter without translocating proximally or distally and especially without becoming separated from the catheter. The stent, particularly its distal and proximal ends, must be protected to prevent distortion of the stent and to prevent abrasion and/or reduce trauma of the vessel walls. It is also important to prevent stent flaring during bending and tracking of the stent.

In light of the above, it would be desirable to employ a stent covering which functions to help retain the stent on the catheter but which could optionally be left on the catheter during stent delivery so as to avoid damaging the stent or causing undesirable movement of the stent during sheath retraction. It would be desirable to provide for a covering which is flexible and which sufficiently covers a stent so as to prevent stent elements from protruding outward from the catheter and interfering with a vessel wall prior to delivery. It would also be desirable to provide a covering which does not increase the profile of the stent delivery catheter beyond its profile without the covering.

Inflation expandable stent delivery and deployment assemblies are known which utilize restraining means that overlie the stent during delivery. U.S. Patent No. 4,950,227 to Savin et al., relates to an inflation expandable stent delivery system in which a sleeve overlaps the distal or proximal margin (or both) of the stent during delivery. During inflation of the stent at the deployment site, the stent margins are freed of the protective sleeve(s). U.S. Patent 5,403,341 to Solar, relates to a stent delivery and deployment assembly which uses retaining sheaths positioned about opposite ends of the compressed stent. The retaining sheaths of Solar are adapted to tear under pressure as the stent is radially expanded, thus releasing the stent from engagement with the sheaths. U.S. Patent No. 5,108,416 to Ryan et al., describes a stent introducer system which uses one or two flexible end caps and an annular socket surrounding the balloon to position the stent during introduction to the deployment site.

Other patents which describe socks or sleeves, and material used therefore include Blaeser et al. U.S. Patent No. 5,944,726 issued 8/31/99; Dusbabek et al. U.S. Patent No. 5,968,069, issued 10/19/99; and Cornelius et al., U.S. Patent No. 6,068,634, issued 5/30/00. In addition, U.S. Patents No. 6,395,008 ; 5,980,530 ; 6,221,097 ; 6,964,676 ; 6,432,130 ; 6,554,841 ; 6,565,595; and 6,607,552 all relate to stent retaining sleeves or socks.

Further examples of stent retaining sleeves are disclosed in U.S. 5,108,416 and WO 01/22903. The latter was published after the priority date of the present invention, and falls under Article 54(3)EPC.

WO 01/22903 discloses a stent delivery system according to the preamble of claim 1 of the present invention, wherein the stent retaining sleeve covers the stent completely.

WO 00/76425 A1, also falling under Article 54(3) EPC, discloses a stent delivery system having a retaining sleeve secured at its distal end in a cuff mounted on the catheter shaft. The retaining sleeve covers the stent completely and may have a plurality of openings.

### BRIEF SUMMARY OF THE INVENTION

This invention provides an improvement over the prior art, by providing a stent delivery system which includes one or more stent retaining sleeves or socks which are constructed and arranged to retract off of the stent when the stent is expanded. The sleeves of the present invention are capable of retaining and immobilizing a non-crimped or crimped stent on the catheter surface by completely covering all of the stent, the sleeves are readily retracted from off of the stent to provide for safe and effective stent release.

The invention may utilize a single sock or sleeve which either retracts proximally or distally, or two sleeves which each cover one end of the stent and retract axially away from each other. The socks or sleeves may be ribbed or accordion; have holes or perforations or be made of two or more materials to construct one or two socks which retract off of the stent when the stent is expanded. The sleeve or sleeves may be composed of an elastic polymer, a non-elastic polymer or a combination thereof.

### BRIEF DESCRIPTION OF THE SEVERAL VIEWS OF THE DRAWINGS

A detailed description of the invention is hereafter described with specific reference being made to the drawings in which:
FIG. 1 is a side view of the stent delivery system showing a single sleeve attached proximally of the stent;
FIG. 2 is a side view of the stent delivery system showing a single sleeve attached distally of the stent;
FIG. 3 is a side view of the stent delivery system showing both a proximal and distal socks, each attached on the proximal and distal sides of the stent, respectively;
FIG. 4 is a side view of the embodiment of FIG. 3 with ribbed socks;
FIG. 5 is a side view of a perforated sock, and
FIG. 6 is a side view of a striped sock.

### DETAILED DESCRIPTION OF THE INVENTION

While this invention may be embodied in many different forms, there are shown in the drawings and described in detail herein specific preferred embodiments of the invention. The present disclosure is an exemplification of the principles of the invention and is not intended to limit the invention to the particular embodiments illustrated.

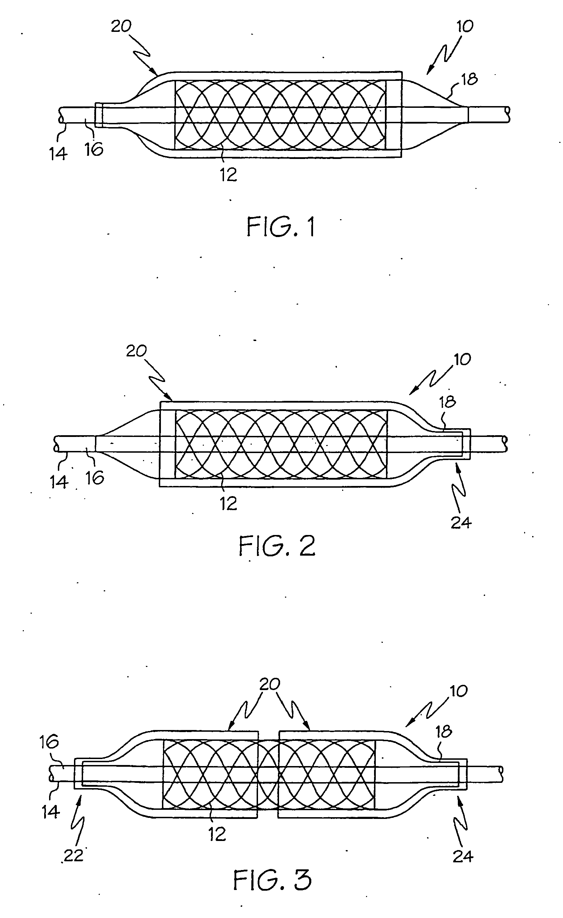

FIG. 1 shows a first embodiment of a stent delivery system, indicated generally at 10, which includes a stent 12 mounted upon a stent delivery catheter 14. FIG. 1 shows the stent delivery system prior to stent delivery. The stent delivery catheter 14 includes a catheter shaft 16 with and inflatable portion or balloon 18. A stent retaining sleeve 20 is engaged at a proximal portion 22 of the catheter shaft 16 adjacent to the balloon 18. The sleeve 20 extends distally over the stent 12 when in the unexpanded position. When the balloon 18 is expanded and the stent 12 expands from the unexpanded state to the expanded state, the sleeve 20 will be retracted proximally off of the stent 12 thereby allowing the stent to deploy.

FIG. 2, on the other hand, depicts the stent delivery system 10 having a sleeve 20 secured to a distal portion 24 of the catheter shaft 16. In the embodiment shown in FIG. 2, the sleeve 20 is retracted distally when the stent 12 is expanded from the unexpanded state to the expanded state.

Fig. 3 shows a stent delivery system which employs a pair of sleeves 20. One of the sleeves 20 is engaged at the proximal portion 22 and one sleeve 20 is engaged to the distal portion 24. According to the present invention, the present sleeves are constructed to cover not only the very ends of the stent 12, but they have sufficient length to overlay the entire stent. The sleeves 20 may be configured to overlap one another.

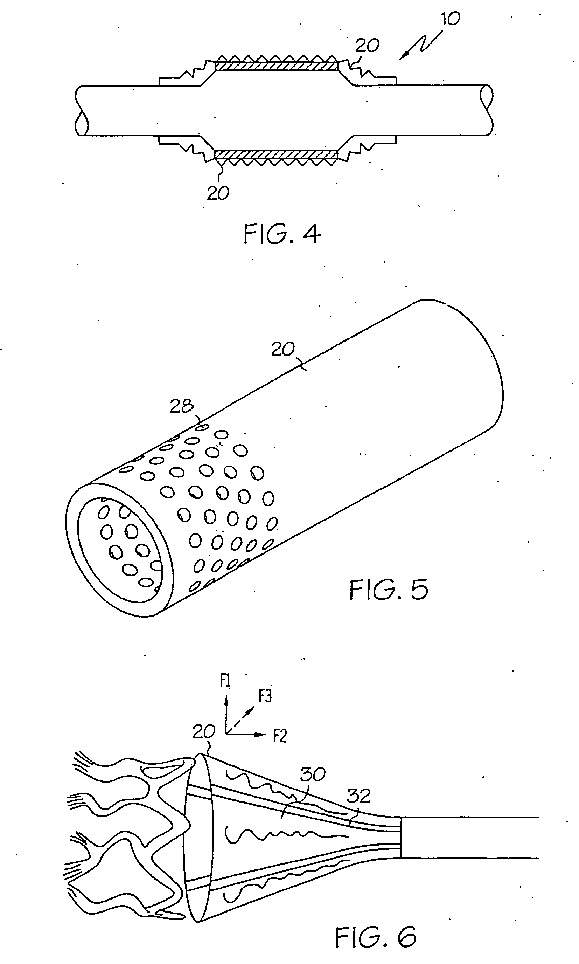

In Fig. 4 an alternative embodiment of the system 10 shown in Fig. 3 is shown. As illustrated in Fig. 4, the sleeves 20 may be provided with an corrugated or ribbed configuration. Such a ribbed configuration may be used for sleeve(s) 20 as shown in Figs. 1-3 to provide the sleeve with the desired characteristics discussed above.

In Fig. 5 another alternative embodiment is shown. In Fig. 5, it may be seen that the sleeve 20 is equipped with a plurality of holes 28. The holes 28 may be provided to the sleeves 20 illustrated in Figs. 1-3 to provide the sleeves 20 with the ability to retract off of the stent as desired.

In Fig. 6 yet another alternative embodiment is shown, wherein the sleeve is composed of a combination of a first material 30 and at least one additional material 32. As illustrated the materials 30 and 32 may be configured in the sleeve 20 to provide a stripped configuration. Such a sleeve may be used in any of the embodiments shown in Figs. 1-3.

The above examples and disclosure are intended to be illustrative and not exhaustive. These examples and description will suggest many variations and alternatives to one of ordinary skill in this art, as included within the scope of the attached claims.

## Claims

1. A stent delivery system comprising:
• a stent delivery catheter (14), the stent delivery catheter having a stent (12) mounting region, the stent mounting region having an inflatable portion;
• a stent (12) disposed about the stent mounting region, the stent having an unexpanded position and an expanded position;
• at least one stent retaining sleeve (20) having first and second ends, the first end being attached to the stent delivery catheter, the at least one stent retaining sleeve disposed about at least a portion of the stent in the unexpanded position, the stent retaining sleeve being constructed and arranged to retract towards the attached first end when the stent is expanded, wherein the second end of the stent retaining sleeve (20) is provided as a free end with respect to the catheter shaft,
**characterized in that**
• the at least one stent retaining sleeve (20) covers the stent completely and has a plurality of openings.

2. The stent delivery system of claim 1 wherein the at least one stent retaining sleeve is ribbed.

3. The stent delivery system of claim 1 wherein the at least one stent retaining sleeve is pleated.

4. The stent delivery system of claim 1 wherein the at least one stent retaining sleeve is comprised of at least two materials.

5. The stent delivery system of claim 4 wherein the at least one stent retaining sleeve is comprised of alternating strips of the at least two materials.

6. The stent delivery system of claim 1 wherein the first end is attached proximally of the stent.

7. The stent delivery system of claim 1 wherein the first end is attached distally of the stent.

8. The stent delivery system of claim 1 wherein the at least one stent retaining sleeve comprises proximal and distal stent retaining sleeves, each having first and second ends, the first end of the proximal stent retaining sleeve being attached to the stent delivery catheter proximally of the stent and the first end of the distal stent retaining sleeve being attached to the stent delivery catheter distally of the stent.

9. The stent delivery system of claim 8 wherein the proximal and distal stent retaining sleeves overlap.

## Patentansprüche

1. Stenteinbringungssystem, umfassend:
• einen Stenteinbringungskatheter (14), wobei der Stenteinbringungskatheter einen Befestigungsbereich für den Stent (12) aufweist, wobei der Befestigungsbereich für den Stent einen füllbaren Abschnitt aufweist;
• einen Stent (12), der um den Befestigungsbereich für den Stents herum angeordnet ist, wobei der Stent eine nicht ausgedehnte Position und eine ausgedehnte Position aufweist;
• mindestens eine Stenthaltehülse (20), die ein erstes und zweites Ende aufweist, wobei das erste Ende an dem Stenteinbringungskatheter befestigt ist, wobei die mindestens eine Stenthaltehülse in der nicht ausgedehnten Position um mindestens einen Abschnitt des Stents angeordnet ist, wobei die Stenthaltehülse so aufgebaut und angeordnet ist, dass sie sich zu dem befestigten ersten Ende hin zurückzieht, wenn der Stent ausgedehnt wird, wobei das zweite Ende der Stenthaltehülse (20) als freies Ende im Verhältnis zum Katheterschaft bereitgestellt ist,
**dadurch gekennzeichnet, dass**
• die mindestens eine Stenthaltehülse (20) den Stent vollständig bedeckt und eine Mehrzahl Öffnungen aufweist.

2. Stenteinbringungssystem nach Anspruch 1, wobei die mindestens eine Stenthaltehülse gerippt ist.

3. Stenteinbringungssystem nach Anspruch 1, wobei die mindestens eine Stenthaltehülse gefaltet ist.

4. Stenteinbringungssystem nach Anspruch 1, wobei die mindestens eine Stenthaltehülse aus mindestens zwei Werkstoffen besteht.

5. Stenteinbringungssystem nach Anspruch 4, wobei die mindestens eine Stenthaltehülse aus einander abwechselnden Streifen aus den mindestens zwei Werkstoffen besteht.

6. Stenteinbringungssystem nach Anspruch 1, wobei das erste Ende proximal am Stent befestigt ist.

7. Stenteinbringungssystem nach Anspruch 1, wobei das erste Ende distal am Stent befestigt ist.

8. Stenteinbringungssystem nach Anspruch 1, wobei die mindestens eine Stenthaltehülse proximale und distale Stenthaltehülsen aufweist, von denen jede erste und zweite Enden aufweist, wobei das erste Ende der proximalen Stenthaltehülse proximal am Stent an dem Stenteinbringungskatheter befestigt ist und das erste Ende der distalen Stenthaltehülse distal am Stent an dem Stenteinbringungskatheter befestigt ist.

9. Stenteinbringungssystem nach Anspruch 8, wobei die proximalen und die distalen Stenthaltehülsen überlappen.

## Revendications

1. Système de fourniture de stents, comprenant :
• Cathéter de fourniture de stents (14), le cathéter de fourniture de stents ayant une région de pose de stents (12), la région de pose de stents ayant une partie gonflable ;
• un stent (12) disposé près de la région de pose de stents, le stent ayant une position non dilatée et une position dilatée ;
• au moins un manchon de retenue de stents (20) ayant des première et deuxième extrémités, la première extrémité étant attachée au cathéter de fourniture de stents, le manchon de retenue de stents, au moins au nombre de un, disposé près d'au moins une partie du stent dans la position non dilatée, le manchon de retenue de stents étant construit et agencé pour se rétracter vers la première extrémité attachée quand le stent est dilaté, où la deuxième extrémité du manchon de retenue de stents (20) est réalisée en tant qu'extrémité libre par rapport à la tige de cathéter,
**caractérisé en ce que**
• le manchon de retenue de stents (20), au moins au nombre de un, couvre le stent complètement et a une pluralité d'ouvertures.

2. Système de fourniture de stents selon la revendication 1, où le manchon de retenue de stents, au moins au nombre de un, est strié.

3. Système de fourniture de stents selon la revendication 1, où le manchon de retenue de stents, au moins au nombre de un, est plissé.

4. Système de fourniture de stents selon la revendication 1, où le manchon de retenue de stents, au moins au nombre de un, est constitué d'au moins deux matériaux.

5. Système de fourniture de stents selon la revendication 4, où le manchon de retenue de stents, au moins au nombre de un, est constitué de bandes alternées des matériaux, au moins au nombre de deux.

6. Système de fourniture de stents selon la revendication 1, où la première extrémité est attachée de façon proximale au stent.

7. Système de fourniture de stents selon la revendication 1, où la première extrémité est attachée de façon distale au stent.

8. Système de fourniture de stents selon la revendication 1, où le manchon de retenue de stents, au moins au nombre de un, comprend des manchons de retenue de stents proximaux et distaux, chacun ayant des première et deuxième extrémités, la première extrémité du manchon de retenue de stents proximal étant attachée au cathéter de fourniture de stents de façon proximale au stent, et la première extrémité du manchon de retenue de stents distal étant attachée au système de fourniture de stents de façon distale au stent.

9. Système de fourniture de stents selon la revendication 8, où les manchons de retenue de stents distaux et proximaux se chevauchent.
